# EUROPEAN PATENT SPECIFICATION

(11) **EP 1 276 437 B1**
(45) Date of publication and mention of the grant of the patent: **10.03.2010**
(21) Application number: 01919723.5
(22) Date of filing: 27.03.2001
(51) Int. Cl.: A61F 2/82

(54) **NARROWING IMPLANT**
VERENGENDES IMPLANTAT
IMPLANT DE RETRECISSEMENT

(30) Priority: 27.03.2000 US 534968
(43) Date of publication of application: 22.01.2003
(73) Proprietor: Neovasc Medical Ltd., Or Yehuda 60376 (IL)
(72) Inventor: SHALEV, Ilan, 53415 Givataim (IL); TSEHORI, Jonathan, 52255 Ramat Gan (IL); DARVISH, Nissim, 30850 Tzrufa (IL)
(74) Representative: van Westenbrugge, Andries
(86) International application number: PCT/IL2001/000284
(87) International publication number: WO 2001/072239

(56) References cited:
- EP-A- 0 779 062
- DE-C- 19 509 464
- US-A- 5 772 668

## Description

### FIELD OF THE INVENTION

The present invention relates to devices for narrowing bodily conduits, for example, blood vessels, to less than their normal diameter.

### BACKGROUND OF THE INVENTION

The heart pumps blood through the body. The heart itself is fed by coronary arteries that end at capillaries. The capillaries are drained by a network of coronary veins, that (typically) flow into a vein known as the coronary sinus. The coronary sinus is a short, large diameter vein that is substantially contiguous with a right atrium, the atrium that collects all venous blood from the body.

Occlusion of coronary arteries is a leading cause of death, especially sudden death, in what is commonly called a "heart attack". When blood flow to a portion of the heart is suddenly stopped, the portion becomes ischemic and its electrical activity is disrupted. As the activity of the heart is mediated by electrical signal propagation, such disruption typically propagates to the rest of the heart, disorganizes the heart's activation and causes the heart output to be reduced drastically, which leads to ischemia and death of the brain. In addition, the disorganized activity often damages the heart beyond what was caused directly by the blockage.

If a patient survives the direct effects of the heart attack, the damage to the heart may predispose the patient to future electrical disorders and/or may significantly reduce the cardiac output, thus reducing quality of life and life expectancy.

Angina pectoris is a chronic or semi-chronic condition which, while not life-threatening, significantly reduces quality of life. In general, the heart responds to increased demand by working harder, requiring more coronary blood flow. When coronary arteries are stenosed or occluded, the increased blood flow cannot be provided, and pain, caused by the resulting ischemia, is produced.

The heart has natural mechanisms to overcome stenosis in coronary arteries. One such mechanism is angiogenesis, in which new arteries are created, for bypassing the stenosis.

Since angiogenesis does not always occur naturally, various procedures have been suggested to encourage it. For example Trans-Myocardial Revascularization (TMR), is a process in which multiple holes are drilled in the heart, with the intent of causing new vessels to be created.

Beck, in "The Surgical Management of Coronary Artery Disease: Background, Rationale, Clinical Experience" by C.S. Beck and B. L. Brofman, by the American College of Physicians in Annals of Internal Medicine Vol. 45, No. 6, December 1956 and in "Long Term Influence of the Beck Operation for Coronary Heart Disease", by B. L. Brofman in the American Journal of Cardiology August 1960, performed open chest surgery in which a coronary sinus vein was restricted, by an external suture. After a few months, coronary blood supply apparently improved. However, this method has fallen in disfavor, in part probably due to the need to open the chest and lift up the heart, to reach the coronary sinus vein.

A standard treatment of stenosed arteries is inserting a stent into the artery, at the stenosed point. The stent, for example a metal coil or mesh, is expanded to have an inner diameter similar to that of the original stenosed blood vessel. If many stenoses are present, it is not common to implant multiple stents. Instead, a bypass procedure, in which a conduit is used to bypass the stenoses, is performed.

US patent 5,618,301, describes a stent-like device for reducing the diameter of a body conduit What is described is an open mesh stent that can be inserted in a stented channel created by a TIPS (Trans-Jugular Intra-Hepatic Portal-Systemic Shunt) procedure, to reduce the blood flow rate through the channel. In order to ensure the flow diameter is reduced and prevent flow through the open mesh, a plurality of thromobogentic threads are provided on the outside of the mesh. However, as can be appreciated, intentionally forming thrombosis in most any part of the vascular system, and especially near the heart, can lead to propagating coagulation or floating thromboses, which are potentially fatal.

German patent DE 195 09 464 describes a vessel implant which can be used as a filter or to close up a blood vessel.

European patent 0 779 062 describes a method for forming a stent.

US patent 5,772,668 describes a stent comprising a continuous loop.

### SUMMARY OF THE INVENTION

The invention relates to a diameter reducing implant adapted for insertion into blood vessels. The implant (reducer) includes at least one narrowed lumen portion, for limiting blood flow. In an exemplary embodiment of the invention, the reducer is designed to not cause blood coagulation outside of the narrowed blood flow. The reduced diameter may, for example, reduce total blood flow through the implant or change the temporal profile of such flow and/or temporal profile of pressure in the vessel.

In an exemplary embodiment of the invention, the reducer is designed not to damage vessel walls, for example, artery walls or vein walls. In one example, the reducer edges are curled. Alternatively or additionally, the reducer edges are coated with a soft coating. Alternatively or additionally, the reducer edges extend parallel to the vessel. Alternatively or additionally, the elasticity of the reducer is low, to prevent undue pressure on the walls.

The blood vessel is a coronary vein or a coronary sinus. Optionally, the narrowing reduces the vessel cross-section by 30%, 50%, 80%, 90% or any other lower, larger or intermediate amount, or even completely occludes the vessel. It should be noted that the heart generally has additional drainage paths besides the coronary sinus, so that even complete occlusion of the coronary sinus will not generally prevent blood from reaching the coronary capillaries. For example, the narrowing may have an inner diameter of 1 mm, 2 mm, 3 mm or any larger, smaller or intermediate size. Optionally, the unexpanding reducer is between 10 mm and 80 mm long. Optionally, the reducer is asymmetric, for example, adapted to fit the normal shape of the coronary sinus.

In an exemplary embodiment of the invention, the reducer includes one or more narrowed sections and one or more un-narrowed sections. In an exemplary embodiment of the invention, narrowing sections are non-expandable, expand less or require a greater force to cause them to expand, as compared to un-narrowed sections. In an exemplary embodiment of the invention, the un-narrowed sections expand a considerable amount, for example a factor of 2, 3, 4 or 5, or any greater, smaller or intermediate factor, in diameter, from their diameter during insertion.

In an exemplary embodiment of the invention, the narrowed sections comprises a ring. Optionally, the ring defines a mesh to allow some expansion thereof. Optionally, after a reducer is deployed, the ring may be further expanded, to reduce the degree of narrowing.

The vessel walls may collapse or are urged to collapsed onto the reducer. Alternatively, a coagulation-encouraging material or threads are provided outside the narrowed portion (i.e., between the portion and the vessel wall) to encourage the formation of clots between the reducer body and the vessel wall.

Optionally, the reducer is coated with a flexible coating (inside and/or out) and/or defines a dense mesh pattern, that prevents or reduces blood flow through the reducer surface, for example, forcing at least 40%, 60%, 80%, 90% or any smaller, greater or intermediate flow percentage to be through an axial lumen defined by said reducer. In an exemplary embodiment of the invention, the dense mesh fills at least 30%, 40%, 60%, 70%, 80% or any greater, smaller or intermediate percentage of a surface of the reducer.

In an exemplary embodiment of the invention, the reducer comprises a rim, which rim is constructed to be more difficult to expand (for plastic) or expand less (for self-expanding) than portions of the reducer just inside the rim. Thus, when the reducer is expanded, the rim does not flare out and an inwards curving or a parallel profile is achieved in the reducer. Optionally, the rim defines a maximal radius of the rim, to prevent over expansion of the rim of the reducer.

In an exemplary embodiment of the invention, the reducer is a plastically deformed reducer, expanded using a balloon. In order to prevent the balloon from catching in the narrowed section of the reducer, the balloon comprises a plurality of fingers on its outside, so that the fingers can bend back and be pulled out through the narrowing. Alternatively or additionally, the fingers are asymmetric, so that when the balloon deflates, the balloon will twist closed.

In a method of installing a narrowing device, the narrowing device is installed in a coronary sinus vein (hereafter "coronary sinus"). Alternatively or additionally, the narrowing device is installed in one or more coronary veins, for example the great coronary vein. In an exemplary embodiment of the invention, a delivery catheter is inserted through a central vein, such as the Jugular vein and brought to the coronary sinus. The reducer is released from the delivery catheter and allowed to elastically expand and/or is plastically expanded using a balloon. Optionally, a pressure sensor is provided on the delivery catheter for assessing the effect of the reducer on the venous pressure before the reducer and/or after the reducer.

In a method of selecting a reducer, functional information on the heart is used to assess need. An image, such as an echo-cardiography image, a Doppler image or a CT image is used to measure the coronary sinus (or any other target vein). The size of the reducer and its degree of narrowing are then selected to match the geometry of the coronary sinus and/or the desired therapeutic effect.

There is thus provided in accordance with an exemplary embodiment of the invention, a reducer for insertion in a blood vessel, comprising:
at least one narrowed section having a first diameter; and
at least one flared section having a diameter at least 20% greater than said first diameter,
   wherein said reducer is formed of a material and has a geometry that does not cause coagulation of blood in its vicinity. Optionally, said reducer is operative to increase a coronary artery blood pressure when inserted in a coronary vein. Optionally, said reducer is operative to modify a coronary artery blood flow distribution when inserted in a coronary vein. Optionally, said reducer is operative to increase a coronary sinus blood pressure when inserted in a coronary sinus. Optionally, said reducer is operative to increase an intra-myocardial perfusion when inserted in a coronary sinus.

In an exemplary embodiment of the invention, said flared section includes at least one area adapted to contact a wall of a vein. Optionally, said area is made large enough to prevent damage to the wall. Optionally, said area has an axial extent of at least 2 mm. Optionally, said area has an axial extent of at least 4 mm.

In an exemplary embodiment of the invention, said flared section has an outside edge. Optionally, said outside edge lies on a single plane. Alternatively or additionally, said outside edge is defined by a plurality of elongate sections that cooperate to define a maximal rim for said reducer. Alternatively or additionally, said outside edge is smooth. Alternatively or additionally, said outside edge is curved inwards towards an axis of said reducer. Alternatively or additionally, said outside edge is coated with a soft material.

Said reducer doesn't cause turbulence inside a lumen defined by said narrowed section and said flared section. Alternatively or additionally, said narrowed section comprises a ring segment having a different surface design from flared section. Alternatively or additionally, said narrowed section comprises a solid ring.

In an exemplary embodiment of the invention, said narrowed section comprises an array of cell elements.

In an exemplary embodiment of the invention, said reducer is suited to be expanded, after insertion, from an unexpanded configuration to an expanded configuration. Optionally, said flared section is plastically deformable to provide said configuration change. Alternatively said flared section is self-expanding to provide said configuration change.

In an exemplary embodiment of the invention, said narrowed section self-expanding to provide said configuration change. Alternatively, said narrowed section is plastically deformable to provide said configuration change. Alternatively, said narrowed section does not expand. Alternatively, said narrowed section is further expandable after said reducer is in said expanded configuration.

In an exemplary embodiment of the invention, said reducer comprises a ring mounted outside of said narrowed section, said ring defining a maximal diameter of said narrowed section.

In an exemplary embodiment of the invention, said narrowed section is formed of a pliable material. Alternatively or additionally, said reducer is formed of at least one of an elastic material, a shape-memory material and a super-elastic material.

In an exemplary embodiment of the invention, different parts of said reducer have different degrees of resistance to deforming. Optionally, said narrowed section has a greater resistance to deformation than said flared section. Alternatively, a rim area of said flared section has a greater resistance to deformation than an adjacent part of said flared section.

In an exemplary embodiment of the invention, said narrowed section has an axial extent of between 1 mm and 5 mm.

In an exemplary embodiment of the invention, said reducer has an axial extent of between 10 mm and 30 mm.

In an exemplary embodiment of the invention, said narrowed section has a cross-sectional area of less than 70% of a maximum cross-sectional area of said flared section. Optionally, said narrowed section has a cross-sectional area of less than 50% of a maximum cross-sectional area of said flared section. Optionally, said narrowed section has a cross-sectional area of less than 40% of a maximum cross-sectional area of said flared section. Optionally, said narrowed section has a cross-sectional area of less than 30% of a maximum cross-sectional area of said flared section. Optionally, said narrowed section has a cross-sectional area of less than 20% of a maximum cross-sectional area of said flared section.

In an exemplary embodiment of the invention, said flared section has an axial extent of between 4 mm and 10 mm.

In an exemplary embodiment of the invention, said reducer is adapted for insertion in a human coronary sinus. Alternatively or additionally, said reducer is adapted for insertion in a human coronary vein. Optionally, said adaptation is by size.

In an exemplary embodiment of the invention, said at least one flared section comprises at least two flared sections.

In an exemplary embodiment of the invention, said reducer describes an hourglass figure.

In an exemplary embodiment of the invention, said flared section is dense, to reduce blood flow therethrough. Alternatively or additionally, said flared section is coated, to reduce blood flow therethrough.

In an exemplary embodiment of the invention, said reducer is formed of a soft material, to reduce contact force against an enclosing vessel wall.

In an exemplary embodiment, said reducer is operative to release a slow release molecule after it is deployed.

In an exemplary embodiment of the invention, said reducer has an outside surface adapted to attach to a wall of a vein.

In an exemplary embodiment of the invention, said narrowed section comprises a valve.

### BRIEF DESCRIPTION OF THE DRAWINGS

Non-limiting embodiments of the invention will be described with reference to the following description of exemplary embodiments, in conjunction with the figures. The figures are generally not shown to scale and any measurements are only meant to be exemplary and not necessarily limiting. In the figures, identical structures, elements or parts which appear in more than one figure are preferably labeled with a same or similar number in all the figures in which they appear, in which:
Fig. 1 is a schematic showing of a reducer installed in a coronary sinus vein, in accordance with an exemplary embodiment of the invention;
Fig. 2 is a schematic side view of a reducer, in accordance with an exemplary embodiment of the invention;
Figs. 3A and 3B illustrate a plan layout of a reducer, in accordance with an exemplary embodiment of the invention;
Fig. 3C shows illustrates the reducer of Fig. 3A in an unexpanded configuration and mounted on a delivery system, in accordance with an exemplary embodiment of the invention;
Fig. 3D illustrates a coil-based reducer, in accordance with an exemplary embodiment of the invention;
Fig. 4A and 4B show a plan layout of a reducer having a smooth rim when expanded, in accordance with an exemplary embodiment of the invention;
Fig. 4C shows a reducer with a smooth rim in an expanded configuration, in accordance with an exemplary embodiment of the invention;
Fig. 5 shows a vascular path to a coronary sinus, in accordance with an exemplary embodiment of the invention;
Fig. 6A shows a plastically deforming reducer delivery system, in accordance with an exemplary embodiment of the invention;
Fig. 6B shows a delivery system for delivering a self-expanding reducer, in accordance with an exemplary embodiment of the invention;
Fig. 6C shows a balloon design, in accordance with an exemplary embodiment of the invention;
Fig. 7 is a flowchart of a method of reducer delivery;
Fig. 8 shows a portion of a plan layout of a section of a reducer with selective narrowing control, in accordance with an exemplary embodiment of the invention; and
Figs. 9A-9F illustrate various reducer variations, in accordance with exemplary embodiments of the invention.

### DETAILED DESCRIPTION OF EXEMPLARY EMBODIMENTS

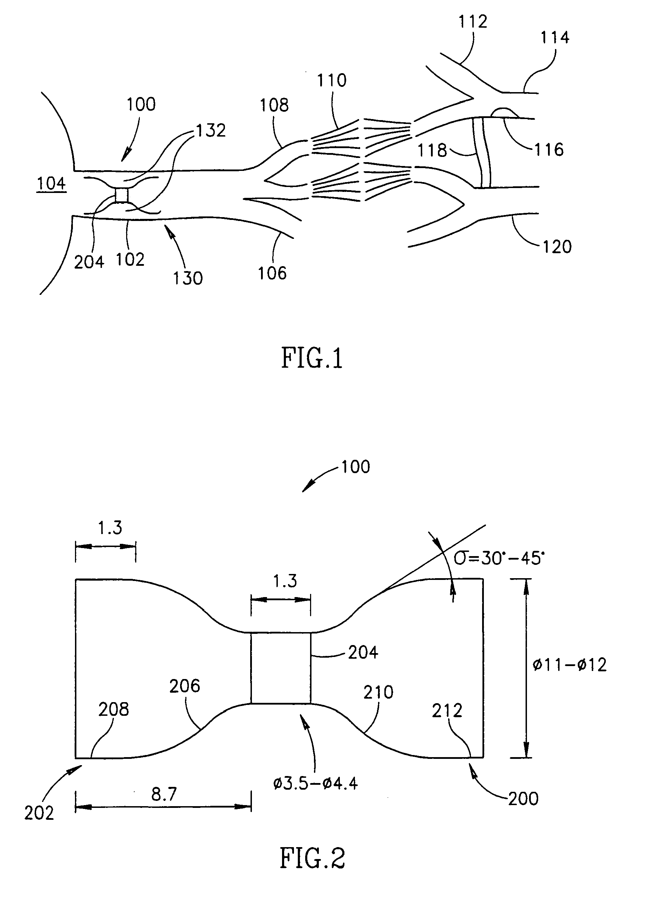

Fig. 1 is a schematic showing of a reducer 100 installed in a coronary sinus vein 102, in accordance with an exemplary embodiment of the invention. Coronary sinus 102 drains a plurality of cardiac veins 106 into a right atrium 104. The cardiac circulation is generally hierarchical and comprises of stages of reducing (or increasing) diameter. Thus, veins 106, in turn, drain a plurality of thin venoules 108, which, after a few stages, drain a plurality of capillaries 110. Capillary 110 are fed by a plurality of arterioles 112, which, after a few stages, are fed by a plurality of coronary arteries 114 and 120. A stenosis 116 is shown in a coronary artery 114. While the cardiac circulation is generally hierarchical, some connection exists between different branches. Occasionally, the existence of stenosis 116 will cause a collateral connection 118 to spontaneously form (or widen an existing connection) between coronaries 114 and 120, bypassing stenosis 116.

In some cases, however, this spontaneous formation does not occur. In an exemplary embodiment of the invention, a reducer 100 is placed in coronary sinus 102 and has a narrowing significant enough to encourage the formation of collateral connection 118. It is hypothesized that collateral connection 118 is caused by an increase in venous blood pressure, which, in turn, increases the pressure in the capillaries and/or causes retro-flow in the capillaries and/or causes drainage of the capillaries directly into the heart. However, even if this hypothesis is incorrect, several studies, that included numerous experiments and actual procedures have shown that constriction of coronary sinus 102 will generally cause the formation of collateral circulation and/or otherwise improve the condition of patients with blocked coronary arteries. Alternative or additional hypotheses which are optionally used to select the constrictive effect of reducer 100 include:
(a) Reducer 100 increases the pressure in the coronary capillaries, thus increasing perfusion duration.
(b) An increase in resistance of the venous system causes redistribution of blood flow in coronary arteries.
(c) An increase in resistance of venous system increases intra-myocardial perfusion pressure and/or intra-myocardial pressure.
(d) Increasing the arterial diastolic pressure (by restricting venous drainage) causes the arterial auto-regulation to start working again, for example, such an auto regulation as described in Braunwald "Heart Disease: A textbook of Cardiovascular Medicine", 5th Edition, 1997, W.B. Saunders Company, Chapter 36, pages 1168-1169.

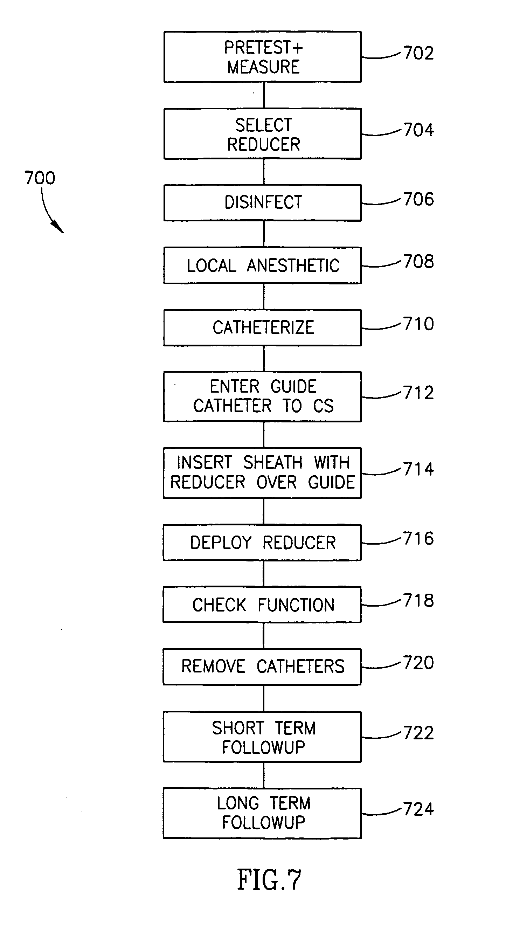

It should be noted that the selection of reducer 100 may be made to achieve one or more of the above suggested effects, optionally to a desired degree and/or taking into account safety issues, such as allowing some drainage and maximum pressure allowed by the coronary venous drainage system. These effects may be determined using various measurements, as described below with reference to Fig. 7.

Fig. 2 is a schematic side view of reducer 100, in accordance with an exemplary embodiment of the invention. Reducer 100 comprises a narrowed section 204 and at least one funnel shaped section 200 (and 202) leading into narrowed section 204. Section 200 (and 202) includes portions 210 and 206 that are inclined relative to the wall of coronary sinus 102 and portions 212 and 208 that are parallel to the wall.

In the exemplary embodiment and measurements shown, reducer 100 is expandable and shortens somewhat during expansion: having a length of 20 mm before expansion and about 18.8 mm after expansion. Optionally, a non-shortening design is used, for example a mesh as in peristaltic stents, such as described in US patent 5,662,713. An exemplary material thickness is 0.15 mm, however, thinner or thicker materials may be used. Other exemplary lengths are 5 mm, 12 mm, 24 mm, 35 mm 45 mm and any smaller, intermediate or larger size. The length is optionally selected to match a physiological size of the target vein (e.g., length and curves) and/or to ensure good contact with vein walls. The length of narrowing 204 may be, for example, 0.5 mm, 1 mm, 2 mm, 3 mm, 5 mm or any smaller, intermediate or larger length, for example selected to achieve desired flow dynamics. An exemplary inner diameter of the flared out sections is between 2 mm and 30 mm, for example, 5 mm, 10 mm, 15 mm, 20 mm or any larger, smaller or intermediate diameter, for example selected to match the vein diameter. The inner diameter of the narrowing may be, for example, 1 mm, 2 mm, 3 mm, 5 mm, 10 mm or any smaller, larger or intermediate diameter, for example selected to achieve desired flow dynamics and/or a pressure differential across the reducer.

In an exemplary embodiment of the invention, the ratio between the cross-section of narrowing 204 and the ends of reducer 100 is 0.9. 0.8, 0.6, 0.4, 0.2 or any larger, smaller or intermediate ratio, for example selected to achieve desired flow dynamics and/or a pressure differential across the reducer.

While a circular cross-section is shown, other cross-sections may be used, for example, polygonal and ellipsoid. A potential advantage of non-circular cross-sections is that the device is less likely to migrate axially. Alternatively or additionally, the outside of the reducer is roughened and/or otherwise adapted to adhere to the vein wall. The cross-section shape and/or orientation optionally changes along the length of reducer 100.

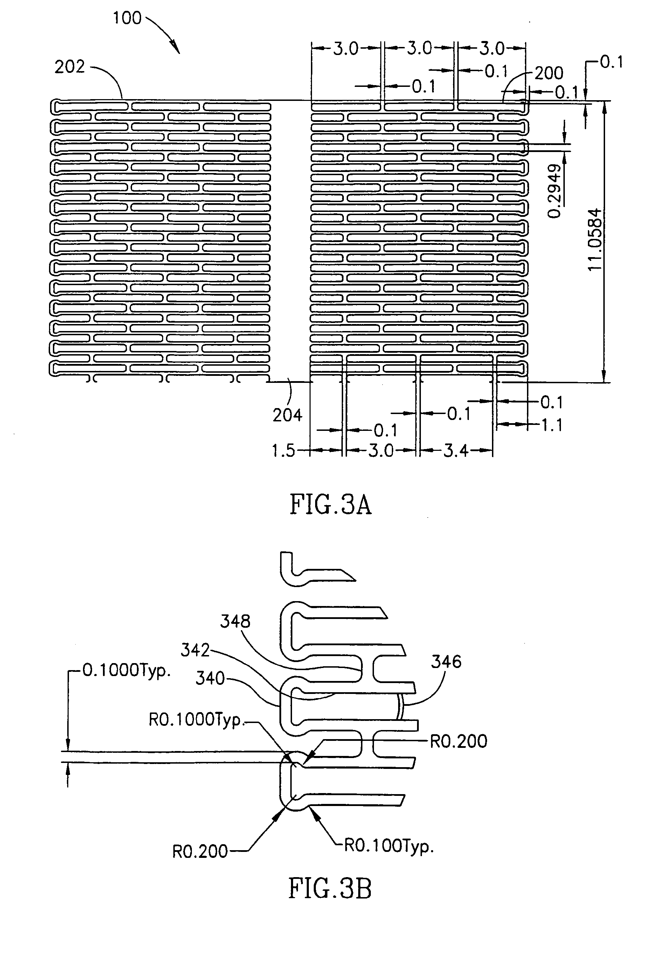

Figs. 3A and 3B illustrate a plan layout of reducer 100, in accordance with an exemplary embodiment of the invention. Fig. 3B shows a detail of the plan layout. In this plan layout, the ends of sections 200 and 202 are caused to be parallel to the vessel wall when reducer 100 is expanded.

In an exemplary embodiment of the invention, the outside rim of reducer 100 is defined by sections 340, 342 and 348, shown in Fig: 3B. Optionally, the total length of these sections defines the maximum rim length. Alternatively or additionally, the bending areas in and between these sections define the relative force required to expand the rim region relative to the area near the rim. If the rim region is more difficult to expand and/or is expanded less than the adjacent regions, the expansion of reducer 100 will tend to cause the rim to bend in, or at least not flare out. Alternatively, in a self-expanding reducer, the existence of sections 340, 342 and 348 can be used to ensure the final shape of the rim. Optionally, additional sections 346 are provided around the circumference of reducer 100, which define outer cells in reducer 100, which outer cells may have a maximum expansion that is the same or smaller than that nearby (axially inwards) cells. This design can also be used to control the shape of the rim.

In an exemplary embodiment of the invention, a reducer is characterized by this maximum diameter, which may be used, for example, for selecting a particular reducer to match a patient. Optionally, during expansion, the balloon is aligned with reducer 100 so that it only contacts the rim region or only contacts the non-rim regions of reducer 100.

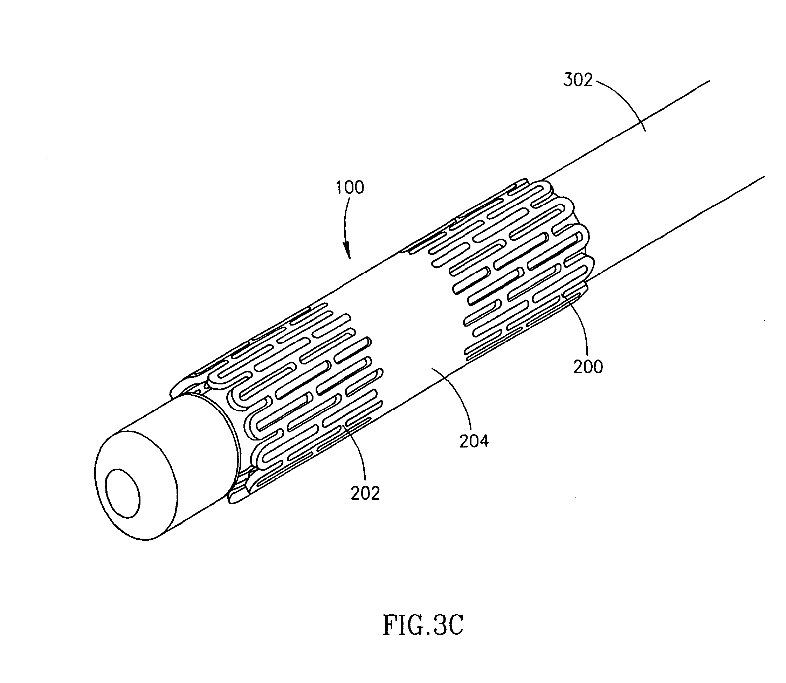

Fig. 3C shows reducer 100 in an unexpanded configuration and mounted on a delivery system 302 (e.g., a balloon catheter).

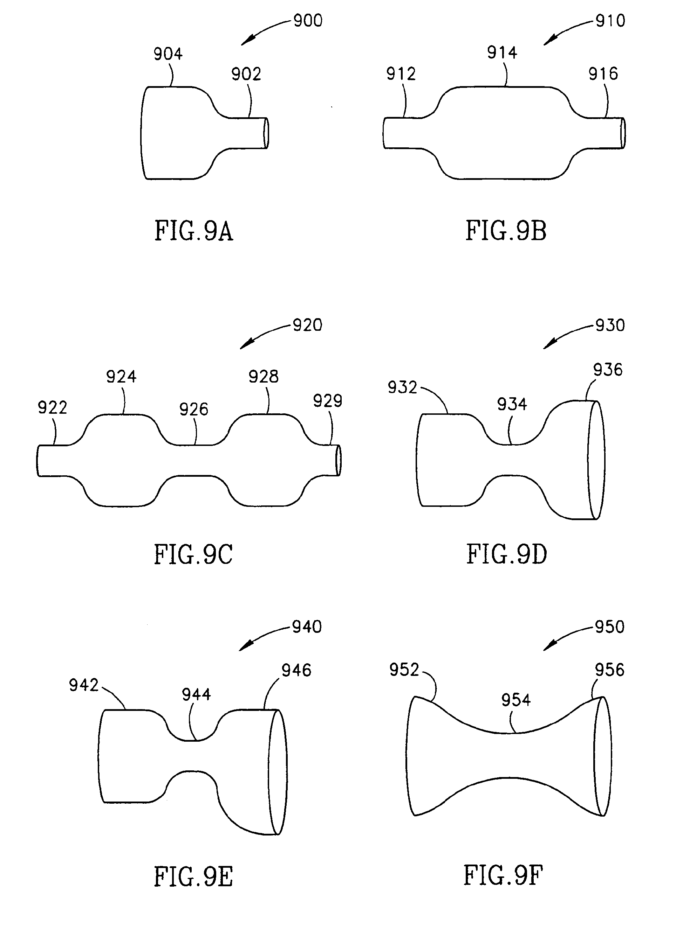

In an exemplary embodiment of the invention, reducer 100 is formed by cutting out of a sheet of metal or a tube, for example, using laser, water cutting, chemical erosion or metal stamping (e.g., with the result being welded to form a tube). Alternatively, reducer 100 is woven (e.g. of metal or plastic fiber), for example, using methods as well known in the art. Optionally, narrowing section 204 is made using a different method from flaring sections 200 and 202, for example, the flaring sections being woven and the narrowing section being cut from sheet metal. In an alternative embodiment of the invention, reducer 100 includes with a constraining ring that prevents the expansion of narrowing section 204. Optionally, the restraining ring is plastically expandable, possibly under a higher pressure than the rest of reducer 100, which may be plastically deformable or self-expanding. Alternatively or additionally, the restraining ring is selected to set the desired degree of narrowing, and then mounted on a reducer, a stent or a stent graft, for implantation. In a sleeve reducer (Fig. 9F, below), a similar effect may be achieved by suturing the stent graft.

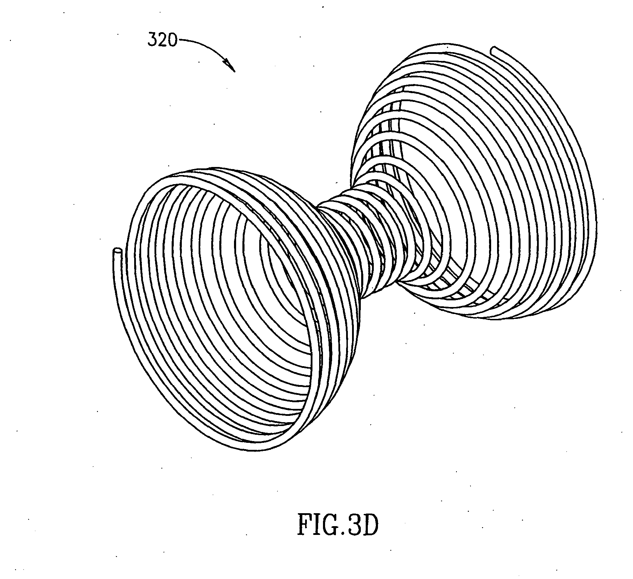

In an alternative embodiment, reducer 100 is cut out of a sheet and then spirally twisted around a mandrel to form the shape of reducer 100. Alternatively, reducer 100 is cut out of a tube, with the flared parts being spiral cuts and the narrowing part being a ring cut. Alternatively, reducer 100 is formed as a coil spring, with axially varying relaxation positions. Fig. 3D illustrates a coil-based reducer 320, in accordance with an exemplary embodiment of the invention.

In an exemplary embodiment of the invention, once reducer 100 is formed, it is mounted in a jig having the desired final expanded shape and heated to train that shape (e.g., for a super-elastic reducer).

In an exemplary embodiment of the invention, reducer 100 is adapted for use in a coronary sinus or other coronary vein. Veins are typified by having a low degree of elasticity and being relatively sensitive to tears (as compared to arteries). In one example, the edges of reducer 100 are curved inwards or curled, for example as shown by reference 130 in Fig. 1. Alternatively or additionally, the edges are folded back and/or smoothed to remove sharp edges. Alternatively, the parallel sections 208 and 212 (Fig. 2) are made long enough to support reducer 100 without harming coronary sinus 102. Alternatively or additionally, reducer 100 or at least larger diameter portions thereof, is made soft enough and/or with a low spring constant, to prevent the reducer from applying too much pressure on the coronary reducer wall. Alternatively or additionally, the ends of reducer 100 are coated with a flexible coating, for example, a soft silicone elastomer or another soft plastic or rubber material such as Latex, Teflon and/or Polyurethane.

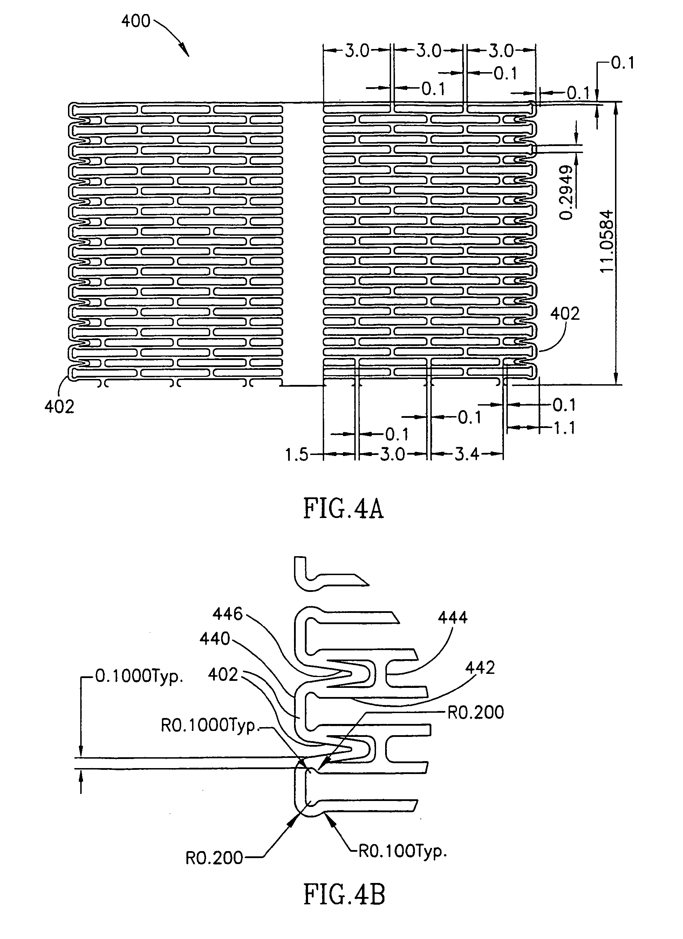

Alternatively or additionally, reducer 100 has a smooth rim at each end. Fig. 4A and 4B show a plan layout of a reducer 400 having a smooth rim 402 (when expanded).

In Fig. 4B, outer rim 402 is defined by sections 440 and 446. As shown, these sections are designed to provide a relative smooth rim, possibly with small amounts of distortion (so rim 402 remains smooth) where the sections connect to sections 442 and 444. Together, sections 442, 444 and 446 define outer cells for rim 402.

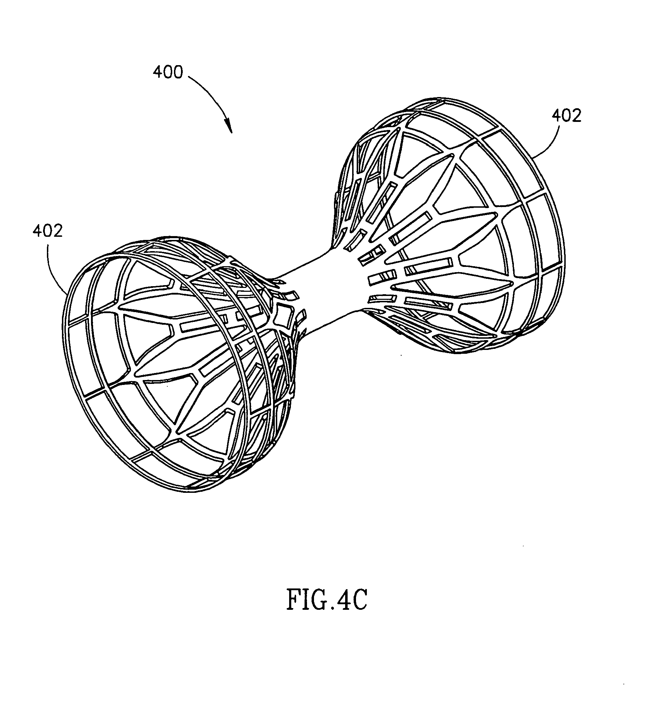

Fig. 4C shows an alternative design for reducer 400, in an expanded configuration, illustrating smooth rims 402.

Referring back to Fig. 1, a region 132 is defined between reducer 100 and the wall of coronary sinus 102. In an exemplary embodiment of the invention, it is desired that little or no blood bypass narrowing 204 through region 132. In some types of reducer 100, this is achieved by sections 200 and 202 being dense enough to slow down blood flow considerably or being practically blood-proof, for example, in a coil-type reducer. Alternatively or additionally, an elastic coating is provided on the inside or outside of reducer 100, for example, latex, to cover and prevent flow through openings in the reducer body. In an exemplary embodiment of the invention, the coating is a separate, flexible layer, that is attached to the reducer at several points (e.g., at the center and at either end, such as to prevent tearing of the layer by the expanding reducer) and is performed to the shape of the expanded reducer, prior to expansion, this coating layer is folded and/or pleated. Alternatively or additionally, one reducer is implanted inside another reducer, with misaligned mesh patterns, so that the solid parts of one reducer block apertures defined by the other reducer.

Alternatively or additionally, the walls of coronary sinus 102 collapse onto reducer 100, blocking any apertures in the body of reducer 100 and preventing flow bypassing narrowing section 204. Optionally, reducer 100 is constructed to encourage such collapsing, for example, by the pattern of apertures in funnel section 200 being different from those in funnel section 202, or by the external diameter of reducer 100 being slightly greater than that of coronary sinus 102. It should be noted that since veins are typically soft and are surrounded by tissue, veins typically collapse when their inner pressure is reduced.

Optionally, the outer surface of reducer 100 includes means for attaching the reducer to the collapsed walls, for example, small barbs, an adhesive and/or a fibrosis-formation encouraging material. Optionally, during implantation of reducer 100, flow of blood from the coronary veins to the reducer is blocked for a short period of time and/or blood in reducer 100 is sucked out, to encourage the coronary sinus walls to collapse onto reducer 100 and attached to reducer 100. Optionally, a reducer used for such a procedure can have very short flaring parts, possible with an outer diameter smaller than that of the coronary sinus.

In some embodiments of the invention, a coagulation enhancing material or geometry (e.g., thrombogenic threads) are provided in area 132, for example, being attached to reducer 100. Thus, region 132 will fill with coagulated blood and prevent further blood flow therethrough. In an exemplary embodiment of the invention, however, the reducer is made as un-thrombogenic as possible (e.g., suitable coatings and geometry, as known in the art), to prevent propagation of clots into the heart. Coagulation enhancing is optionally provided if the reducer is relatively impervious to blood flow through its walls, so that clots are not expected to propagate.

In an exemplary embodiment of the invention, reducer 100 is formed of metal, for example, a NiTi alloy (e.g., Nitinol) or stainless steel (e.g., 316L and 316LS). Alternatively, reducer 100 is formed of- or coated with- other bio-compatible materials, such as Nylon and other plastics. Optionally, reducer 100 is bio-absorbable. A reducer made of plastic can be, for example, cast or injection molded. Depending on the type of material and the processing applied, reducer 100 may be plastically deformable to the geometry shown in Fig. 1. Alternatively, reducer 100 may relax to that geometry, for example, using an elastic, shape-memory or super-elastic relaxation process.

Optionally, reducer 100 is formed of two or more materials, for example, narrowing section 204 being a ring formed of plastic and the flared sections being formed of metal.

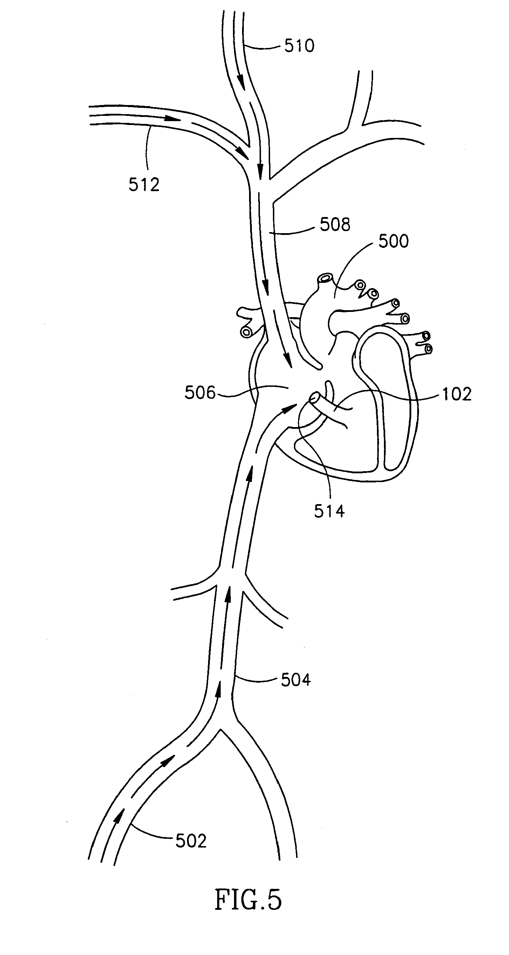

Fig. 5 shows a vascular path to coronary sinus 102, in accordance with an exemplary embodiment of the invention. Desirably, reducer 100 is implanted using a trans-vascular approach, for example, from the venous system or by crossing through an intra-chamber wall in the heart. In an exemplary embodiment of the invention, the delivery system is inserted through a jugular vein 510 or a subclavian vein 512 to a right atrium 506 of a heart 500 via a superior vena cava 508 and/or a femoral vein 502, via an inferior vena cava 504. Once in right atrium 506, the delivery system is guided (e.g., through a sharp bend) to an opening 514 into coronary sinus 102. In some patients, a valve exists at the entrance to coronary sinus 102.

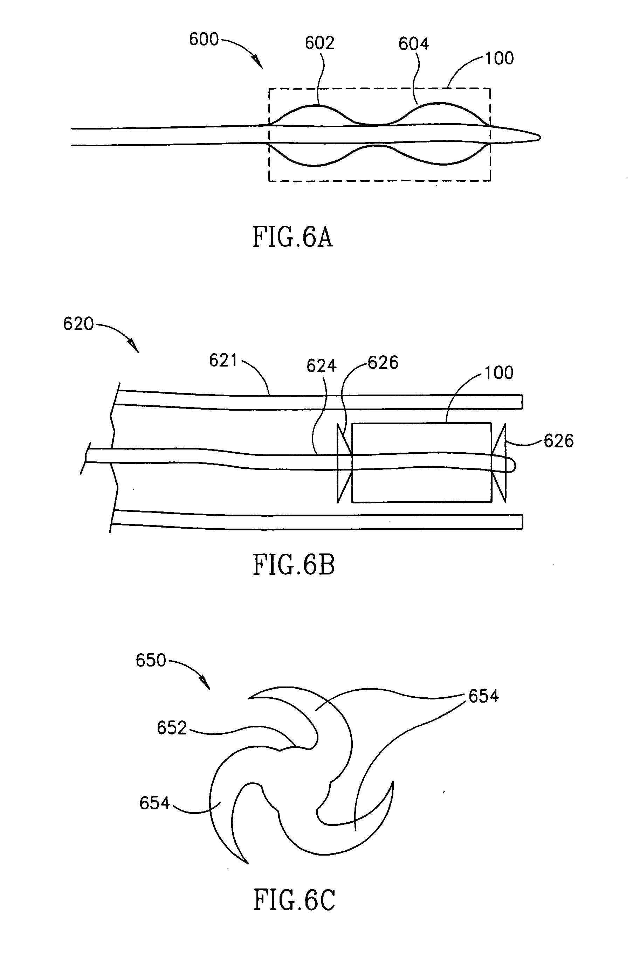

As noted above, reducer 100 can be, for example, actively deformed to the implanted configuration or allowed to relax to the configuration. Fig. 6A shows a plastically deforming reducer delivery system 600, in accordance with an exemplary embodiment of the invention. System 600 can be a standard delivery system designed for deploying a stent over a balloon. Possibly, however, specialized guide sheaths, adapted to the particular path in the right atrium, will be provided. In an exemplary embodiment of the invention, narrowing section 204 of reducer 100 is non-expandable or is less easily expandable, so that the balloon will not completely inflate under narrowing section 204. Alternatively, a non-standard balloon is used, for example, a double balloon, comprising a first balloon section 602 and a second balloon section 604, each adapted to expand one funnel section of reducer 100. Optionally, a single balloon or two balloons are used for this balloon design. Alternatively, in an exemplary embodiment of the invention, a single balloon, that is moved axially from one part of the reducer to another, is used. Alternatively or additionally, after a two-section balloon is used it is replaced with a single section balloon, for example, for selectively expanding a flared end or a narrowing of reducer 100.

Fig. 6B shows a delivery system 620 for delivering a self-expanding reducer, in accordance with an exemplary embodiment of the invention. System 620 can be a system generally like that used for standard self-expanding reducers, for example, including an outer sheath 622 and an inner mandrel 624 on which reducer 100 is mounted. In an exemplary embodiment of the invention, when reducer 100 expands, its inner diameter is greater than the outer diameter of mandrel 624, allowing mandrel 624 to be retracted. In an exemplary embodiment of the invention, mandrel 624 comprises two optional end pieces 626 that define between them a depression, in which reducer 100 is held. End pieces 626 prevent axial migration of reducer 100.

Optionally, the delivery system includes a pressure transducer at its end, for example, for measuring base-line pressure in the coronary sinus. Alternatively or additionally, the delivery system includes a contrast injection channel, for example, for assisting in imaging the coronary sinus before, during and/or after deployment of reducer 100.

Fig. 7 is a flowchart 700 of a method of reducer delivery. It should be appreciated that the procedure of Fig. 7 is an exemplary procedure and other procedures may be applied as well. In particular, the process of Fig. 7 assumes, for clarity, the use of a particular sheath-based delivery system, the use of which is not an essential feature of the invention.

The coronary sinus treatment is combined with an arterial treatment, such as PCTA, stenosis removal (e.g., laser ablation) and/or stenting. The arterial treatment may be applied, for example, before, during or after the venous treatment, possibly during a same use of the catheterization facilities.

At 702, various pre-implantation tests and procedures are optionally applied to a patient to be catheterized, for example, a few weeks, a few days or a few hours before the catheterization. Such procedures can include, for example, one or more of, tests typically applied prior to catheterization and/or reducer delivery, various cardiac function measurements, determination that the patient suffers from ischemic heart disease, determination of angina class, performing electrocardiographgy, full blood work, functional and/or perfusion mapping (e.g., using nuclear medicine imaging techniques such as PET, Thallium or Technetium), to determine pre-procedure perfusion state, echo-cardiography, echo-dobutamin, estimation of micro-cardiological perfusion, Millar catheterization and physiological measurements, such as cardiac output, pulse pressure, left ventricular end-diastolic pressure and stroke volume, left arterial pressure, SVO₂% in the right atrium and/or coronary sinus, intra-myocardial pressure and/or stress testing, such as tread-mill exercise testing. Optionally an imaging technique (e.g., ultrasound, MRI, angiography, or CT) is used to determine the size and/or shape of the coronary sinus and/or other coronary veins.

Just prior to the catheterization, the patient is optionally attached to various monitoring equipment, for example, one or more of ECG (especially to detect and/or monitor one or more of heart rate, ischemic changes, rate disturbances and/or ST segment changes), an arterial line for measuring blood pressure, a pulse oxymeter, a body thermometer and/or apparatus for tracking blood gases. In an optional step 704, a determination is made of desirable properties of reducer 100 and a reducer and/or an expansion protocol is selected to reach these properties. Alternatively, step 704 is performed after catheterization (712). In an exemplary embodiment of the invention, the selection of the reducer depends on one or more of:
(a) coronary sinus length and diameter (e.g., to obtain a matching reducer geometry);
(b) coronary sinus change in diameter near the right atrium (e.g., to obtain a matching reducer geometry);
(c) desired increase in coronary sinus pressure before reducer, optionally including a maximum allowed pressure, for example, 50 mmHg at which coronary sinus is expected to be damaged and/or fail (e.g., to decide what narrowing to select);
(d) desired narrowing (e.g., to decide what narrowing to select);
(e) desired later further narrowing (e.g., to decide on reducer type);
(f) resistance of coronary sinus wall (e.g., how elastic or stiff should reducer be and/or what inflation pressure to use);
(g) desired redistribution of coronary blood flow;
(h) desired retro-flow of blood in coronary arteries; and/or
(i) desired reduction in backflow during right atrium contraction.

The venous location of the reducer is selected to match various cardiac conditions, such as arterial blockage, alternatively or additionally to selecting the reducing diameter for each such reducer. A reducer is implanted in a coronary vein corresponding to a blocked coronary artery. Alternatively or additionally, the locations of implantation are selected to achieve a desired redistribution of coronary artery pressures and/or blood flow, for example, to increase perfusion of ischemic or hibernating portions of the heart.

A database is maintained, which provides a correlation between one or more disease state parameters, the degree of narrowing and/or other reducer parameters, various expansion protocols and/or the expected side effects and/or beneficial effects of the reducer. This database is optionally used to assist in selecting which reducer to use.

At 706, the insertion site is disinfected, for example, near a Jugular vein or a Femoral vein. Depending on the diameter of the delivery system, a smaller vein may be used, possibly allowing the procedure to be less invasive. At 708, a local anesthetic is optionally applied. At 710, a port is inserted in to the vein. Various tests, for example as described above may be performed, for example, one or more of Atrium and chamber pressures, LVEDP, functional tests such as echo-dobutamin and/or perfusion tests. Optionally, Heparin is provided and an ACT (Activated Clotting time) test is performed.

At 712, a guide catheter is inserted into the coronary sinus. In some delivery methods, no separate guide catheter is needed. Before inserting the reducer, various measurements may be performed, for example, a base-line coronary sinus pressure (e.g., using a pressure transducer at the end of the catheter), an angiographic mapping of the coronary sinus, for example to assist in determining what size reducer to use and/or a test obstruction of the coronary sinus, for example to assist in determining a desired narrowing dimension of the reducer that will achieve a desired pressure increase and/or to detect possible side effects in the patient of such a pressure increase.

At 714, a guide sheath is inserted over the catheter to the coronary sinus. However, alternative insertion methods can be used, for example, guiding reducer 100 over a guide wire or along a monorail guide wire. The reducer may be provided with the sheath or it may be inserted through the sheath after the sheath is in place. The reducer may be guided to the coronary sinus. Alternatively or additionally, a suitably sized reducer may be inserted in one or more coronary veins.

At 716, the reducer is deployed. Deployment comprises delivering the reducer and expanding the reducer (e.g., self-expanding or balloon-expanded).

At 718, various measurements are optionally performed, for example, coronary sinus pressures and cardiac functions (e.g., as noted above). Optionally, as described below, the narrowing diameter is changed in response to results of the measuring. Optionally, an image is acquired to ensure the reducer is positioned correctly. Optionally, a measurement of the coronary sinus pressure is made to ensure that the resulting increase in pressure (e.g., by 20 mmHg or 30 mmHg) does not go beyond the holding capacity of the vein or some other safety number (e.g., 50 mmHg). If the pressure exceeds the holding capacity, the narrowing may be enlarged, to reduce the pressure differential. It should be noted that such measurements may be performed before, during and/or after a corresponding arterial treatment that may be performed concurrently with the venous treatment.

At 720, the delivery system is retracted. Optionally, various measurements (e.g., cardiac function) are performed a short-time after deployment, for example, after half an hour.

It is expected that one or more of the following effects is detected (at once and possibly to a greater extent after some delay): retrograde increase in coronary sinus pressure, with a possible associated retrograde flow, improvement of perfusion in some ischemic areas, reduction in venous O₂ saturation (e.g., greater extraction of Oxygen by the cardiac muscle) and/or increase of intra-myocardial pressure, as an indication of possible redistribution of blood supply in the heart. Alternatively or additionally, functional improvements may be viewed, for example, an improvement in segmental contraction, which can be seen using ECHO methods.

At 722, an optional short term follow-up, for example after a few hours, days or weeks is performed. At 724, an optional long term follow-up, for example, after a few months or years is performed. In an exemplary embodiment of the invention, follow-ups are performed after one week, two weeks one month, three months, six months and then yearly. Such follow-up may include, for example, tracking of angina class, treadmill stress test, perfusion estimation (e.g., using SPECT), functional estimation, for example, using echo-dobutamin and/or any other typically applied tests.

It is expected that after a few weeks, the myocardial perfusion and intra-myocardial pressure will increase and redistribution of myocardial blood flow will improve, even beyond the immediate result of the insertion of reducer 100. Possibly, the auto-regulation mechanism of the coronary flow will start working again, by the pressure in the coronary arteries increasing beyond the threshold for activation of the autoregulation mechanism and/or revascularization should start. After a few months, revascularization is expected to be well established, and significantly improve the clinical picture.

Optionally, the above procedure is varied by first placing a stent or a graft into the coronary sinus and mounting the reducer inside the stent or the graft.

Optionally, the reducer includes an integral blood pressure sensor, or a separate small blood pressure monitor is implanted, for example as described in US patent 6,053,873, in WO 00/32092, in WO 99/34731 and in US patent 6,159,156. One or more transducers are optionally implanted, for example, to measure a pressure differential across the reducer.

Reducer 100 may be varied in various manners. It should be noted, that when placing a reducer in a blood vessel it is generally desirable that the flow through the reducer be smooth. Alternatively, the flow may be made turbulent, for the express purpose of reducing the flow rate through the reducer.

Reducer 100 has been described generally as including sections which do not reduce blood flow and sections which do reduce blood flow. In an alternative embodiment of the invention, the entire reducer reduces blood flow, for example, reducing diameter, by at least 20%, 30%, 50% or more. The vein is actively collapsed on the reducer, so that the vein is engaged by barbs on the outside of reducer 100. Optionally, a flared shape is maintained for this version of the reducer, so that flow remains smooth.

Occasionally, it is not possible to insert a reducer with exactly the right diameter. One possible solution is to insert a second reducer, with a smaller diameter in series (e.g., in coronary sinus or other coronary veins) or inside an existing reducer. A reducer for installation inside a second reducer may have a non-standard shape, for example, including only one funnel section 200 (e.g., to prevent the need for the delivery system to pass through the narrowing in the existing reducer) or including ends adapted to engage the reducer rather than to prevent damage to the coronary sinus. Another possible solution is to insert a reducer with a small inner diameter and to increase the diameter as needed, e.g., by progressively inflating a balloon therethrough.

Fig. 6C shows a cross-section through fingered balloon 650, comprising a body 652 and a plurality of fingers 654. When the balloon is deflated, the shape of the fingers and their elasticity causes the fingers to wrap down and reduce the diameter of the balloon. This may prevent the balloon from catching on narrowing 204. Fingers 654 may be axially elongate. Alternatively, the fingers are axially short, possibly with a plurality of axially displaced fingers provided. Such fingers may bend out of the way when the balloon is retracted.

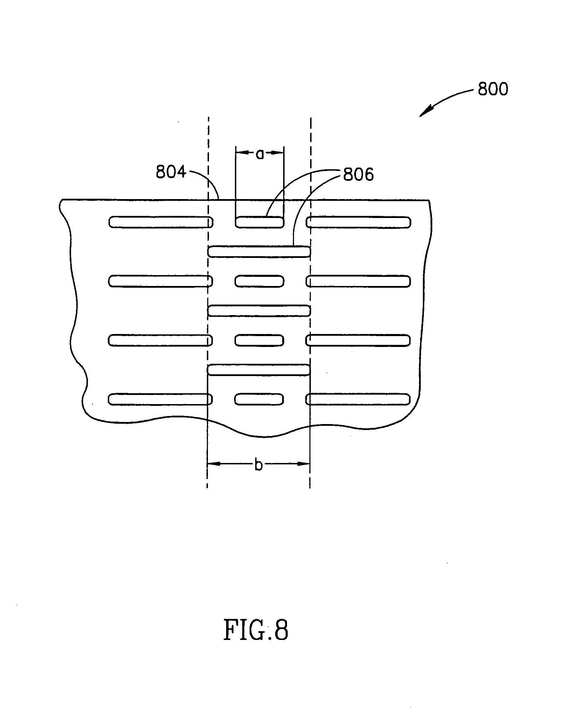

Fig. 8 shows a portion of a plan layout of a section of a reducer 800 with selective narrowing control, in accordance with an exemplary embodiment of the invention. Reducer 800 includes a narrowing section 804. However, section 804 is also expandable, for example, having a plurality of thin slits 806 defined therein. This allows the minimum diameter of reducer 800 to be increased after deployment. In an exemplary embodiment of the invention, section 804 is stiffer than the rest of reducer 800, so that pressure suitable for expanding reducer 800 will not expand section 804. Alternatively, reducer 800 is a self-deploying device and section 804 is plastically deformed using a balloon. Thus, a delivery system used for reducer 800 may include both a restraining element and a balloon element. In case the implantation of a reducer fails, extreme expansion of section 804 will substantially negate the function of reducer 800 and will allow a new reducer to be implanted within reducer 800, at a later time.

Alternatively, as shown, two sizes of slits 806 are provided, with the degree of resistance to deformation being determined by the sizes and/or relative sizes of the slits.

Figs. 9A-9F illustrate various reducer variations, in accordance with exemplary embodiments of the invention. While a sigmoid-like flaring is shown, a linear or other flaring design may also be provided.

Fig. 9A shows a reducer 900 with having a narrowing 902 and only a single flared out portion 904. Narrowing 902 may point upstream or down stream. One potential advantage of this design, is that the delivery system is less likely to get caught inside narrowing 902. Another potential advantage is that a completely obstructing implant can be provided. In an exemplary embodiment of the invention, however, even such a completely obstructing implant has smooth sides, to prevent damage to the coronary sinus. Possibly, the outer diameter of the completely obstructing implant or a nearly complete reducer is increased beyond that of the coronary sinus, to prevent dislodgment of the implant. Alternatively or additionally, one or more barbs on the outside of the implant may be provided. Optionally, a cone shaped reducer is provided with one or more openings for blood flow on the face of the cone, rather than at its apex as shown.

Alternately to a plain reducer, the narrowing may be a valve, for example, a valve which opens, to a full or partial diameter, after a suitable pressure is achieved in the coronary sinus distal from the right atrium. For example, a leaflet valve or other type of vascular valve as known in the heart may be provided.

Fig. 9B shows an alternative reducer 910, with two narrowings 912 and 916 sandwiching a flared out portion 914 between them. Optionally, the different narrowings have a different inner diameter. Optionally, the narrowings are selectively expanded using a balloon to achieve a desired pressure profile.

Fig. 9C shows an alternative reducer 920 with three narrowings 922, 926 and 929 and two flared out portions 924 and 928 between the narrowings.

Fig. 9D is an example of an asymmetric reducer 930, in which one flared out portion 932 has a smaller diameter than a second flared out portion 936, but larger than an intermediate narrowing portion 934. Such a reducer may be useful, for example, for veins that change in size along their length, such as the coronary sinus right next to the right atrium.

In Fig. 9E, a reducer 940 is not symmetric around its axis, with one flared out portion 946 being distorted relative to an axis defined by a second flared out portion 942 and a narrowing portion 944.

Optionally, the reducer is curved. In an exemplary embodiment of the invention, asymmetric or curved reducers include special markings, for example, radio-opaque or radio-transparent areas, to assist correct orientation of the reducer in a blood vessel.

Fig. 9F shows a reducer 950, in which a narrowing section 954 is a sleeve, for example, formed of a flexible graft material, such as Dacron or GoreTex. Reducer 950 further comprises at least one of two outer rings 952 and 956 that serve to anchor reducer 950 in the blood vessel. A potential advantage of using a sleeve is that it can bend to conform to the vein geometry and/or dynamics. Other reducer designs can also bend. Optionally, the graft material is elastic, so it can serve as a pressure limiting valve, to better control coronary sinus pressure. Optionally, a constraining ring is provided on the outside of section 954, to restrict the lumen of reducer 950. Optionally, the ring is placed on reducer 950 during the procedure, to achieve a desired narrowing effect. Alternatively or additionally, the ring is expandable, for example using a balloon, to allow controlling the narrowing of reducer 950. Optionally, the ring is sutured to narrowing section 954. Optionally, section 954 is stiffened, for example, using a wire, as known in the art of stent-grafts.

Optionally, the above various reducers use a delivery system with a matched balloon form (e.g., different diameters of inflation at different parts). Alternatively, a single balloon with controllable inflation (e.g., a central part and a plurality of fingers) is provide, in which the balloon is placed inside the reducer at a certain axial position and inflated to further expand that section. Alternatively, different axial sections of the reducer have different resistance, elasticity, plasticity and/or other mechanical properties.

Optionally, the reducer is adapted to release one or more molecules into the blood flow, for example, angiogenesis causing molecules, adhesion enhancing molecules (e.g., for adhesion to the vein wall), anti-coagulation drugs, growth factors, DNA carriers (e.g., liposomes, plasmids), hormones, and/or other molecules as known in the art. In an exemplary embodiment of the invention, the release is towards the vessel wall and/or towards the blood flow. Optionally, the release is selectively in the part of the flow before the narrowing or after the narrowing, to take advantage or to avoid backflow caused by the reducer. Various release mechanisms are known in the rat and may be used, for example, using a coated reducer, a porous reducer, a reducer with a drug chamber or a reducer that includes a channel between the reducer and the vein wall for holding the drug.

A reducer, similar to that of Fig. 3, has been tested on animals (pigs), using the following procedure.
(a) Anti-Trandelburg of the animal, to increase venous return in the Jugular.
(b) Clearing an area over a vein (e.g., Jugular, Femoral, Subclavian).
(c) Insert F6 sheath directly into vein.
(d) Provide heparin 2500 units.
(e) Insert a catheter to the coronary sinus.
(f) Insert Amplaz super stiff guide of 180 mm or 200 mm to coronary sinus.
(g) Attempt to insert 3.5 mm outer diameter reducer through an IVC filter sheath.
(h) Pass a balloon through a 40 cm or 60 cm arrow sheath inflate and deflate twice and then place reducer on the deflated balloon, and check for stability.
(i) Insert IVC filter sheath to coronary sinus, as distal as possible and pass reducer through sheath. If reducer moves, inflate balloon a bit. Then position reducer in coronary sinus.
(j) Check ACT.
(k) Advance reducer so that it leaves sheath arrow and inflate balloon to fix reducer in place.
(l) Remove balloon (possibly sucking out fluid from balloon using a syringe). Twist the balloon.
(m) If arrow sheath gets stuck on narrowing section of reducer, try rotating sheath and/or using IVC filter sheath as a contra.
(n) If reducer did not fit in step (g), put in a peel away banana sheath.
(o) Insert reducer of (h) through banana sheath. Since banana is 14F and sheath arrow is 10F, there may be some bleeding. Inflate balloon if reducer moves (1F = 0,3 mm).
(p) Do (i)-(1)
(q) Is sheath arrow gets stuck in narrowing section of reducer, take out banana sheath, cut proximal end of balloon and sheath arrow and insert IVC filter sheath for use as a contra (as in (m).

The effect of inserting reducer 100 in four animals (pigs), was estimated to be an average increase in coronary sinus mean pressure from 7.0 mmHg to 24.6 mmHg. These measurements were made with a Swan-Ganz catheter including a narrowing balloon mounted on the catheter to emulate the effect of reducer 100. The amount of narrowing was estimated to be between 70% and 80% based on an angiogram using injected contrast material. In another four pigs, a reducer was implanted the reducer was test implanted and in three pigs, the reducer was implanted.

Two of the pigs in which the device was implanted were sacrificed for assessment of angiogenesis after two and three months. The cardiac tissue was fixed using Formalin. Both pigs showed significant proliferation of small-medium sized vessels containing smooth muscle, which represent coronary collaterals. This was true of almost all the samples, from various parts of the heart, including samples form anterior and mid-posterior wall. The most significant proliferation was evident at the peri-coronary sinus specimens, both anterior and posterior.

While the above has been described for use in coronary veins, a reducer with similar design may also be used in other veins, for example, popliteal, tibial or saphenous veins. In an exemplary embodiment of the invention, one or more reducers are implanted in popliteal veins, to increase back-pressure and possibly enhance tissue perfusion pressure and/or redistribute blood flow in the leg. It is expected that pooling will not occur due to the existence of alternative drainage paths in the leg.

In another example, the reducer can be adapted to match other ducts or conduits in the body, for example, with respect to size, length, degree of narrowing, degree of elasticity and form of contact with the conduit walls.

In an exemplary embodiment of the invention, reducer 100 is provided in kit form, possibly with additional reducers, and including instructions for use and/or size markings. Optionally, the reducer is provided inserted into a delivery system or packaged with a delivery system.

It will be appreciated that the above described methods of deploying a reducer implant may be varied in many ways, including, changing the order of acts, which acts are performed more often and which less often, the type and order of tools used and/or the particular timing sequences used. Further, the location of various elements may be switched. In addition, a multiplicity of various features, both of methods and of devices have been described. It should be appreciated that different features may be combined in different ways. In particular, not all the features shown above in a particular embodiment are necessary in every similar exemplary embodiment of the invention. Further, combinations of features from different embodiments into a single embodiment or a single feature are also considered to be within the scope of some exemplary embodiments of the invention. In addition, some of the features of the invention described herein may be adapted for use with prior art devices, in accordance with other exemplary embodiments of the invention. The particular geometric forms and measurements used to illustrate the invention should not be considered limiting the invention in its broadest aspect to only those forms. Although some limitations are described only as method or apparatus limitations, the scope of the invention also includes apparatus designed to carry out the methods and methods of using the apparatus.

Measurements are provided to serve only as exemplary measurements for particular cases, the exact measurements applied will vary depending on the application.

It will be appreciated by a person skilled in the art that the present invention is not limited by what has thus far been described. Rather, the scope of the present invention is limited only by the following claims.

## Claims

1. A reducer (100) for insertion in a blood vessel, comprising: at least one narrowed section (204) defining an axial lumen, allowing flow of blood therethrough, having a first diameter; and at least one flared section (200), wherein said reducer is adapted to be deformed from a first configuration in which said reducer is unexpanded to the expanded configuration, **characterized in that** said at least one flared section has a largest diameter at least 20% greater than said first diameter, in an expanded configuration, and said at least one narrowed section has an inner diameter and a length suited to achieve desired flow dynamics and/or pressure differential across the reducer.

2. A reducer according to claim 1, wherein the largest diameter of the at least one flared section has a size sufficient to occupy substantially an entire cross-section of a coronary vein of an adult.

3. A reducer according to claim 1 or claim 2, wherein the largest diameter of the at least one flared section has a size sufficient to occupy substantially an entire cross-section of a coronary sinus of an adult.

4. A reducer according to any of claims 1-3, wherein said reducer is adapted to increase an intra-myocardial perfusion when inserted in a coronary sinus.

5. A reducer according to any of claims 1-4, wherein said flared section includes at least one area (208) adapted to contact a wall of a vein.

6. A reducer according to claim 5, wherein said area is made large enough to prevent damage to the wall.

7. A reducer according to any of claims 1-6, wherein said flared section has an outside edge (402).

8. A reducer according to claim 7, wherein said outside edge lies on a single plane.

9. A reducer according to claim 7 or claim 8, wherein said outside edge is defined by a plurality of elongate sections (340, 342, 348)/(440, 446) that cooperate to define a maximal rim 402 for said reducer.

10. A reducer according to any of claims 7-9, wherein said outside edge is smooth.

11. A reducer according to any of claims 7-10, wherein said outside edge is curved inwards towards an axis of said reducer.

12. A reducer according to any of claims 7-11, wherein said outside edge is coated with a soft material.

13. A reducer according to any of the preceding claims, wherein said narrowed section comprises a ring segment having a different surface design from flared section.

14. A reducer according to any of the preceding claims, wherein said narrowed section comprises a solid ring.

15. A reducer according to any of the preceding claims, wherein said narrowed section comprises an array of cell elements.

16. A reducer according to any of claims 1-15, wherein said flared section is plastically deformable to said expanded configuration.

17. A reducer according to any of claims 1-15, wherein said flared section is self-expanding to said expanded configuration.

18. A reducer according to any of claims 1-17, wherein said narrowed section is adapted to self-expand to said expanded configuration.

19. A reducer according to any of claims 1-18, wherein said narrowed section is plastically deformable to said expanded configuration.

20. A reducer according to any of claims 1-18, wherein said narrowed section is adapted not to expand when the reducer expands from the first configuration to the expanded configuration.

21. A reducer according to any of claims 1-20, wherein said narrowed section is further expandable after said reducer is in said expanded configuration.

22. A reducer according to any of claims 1-21, comprising a ring mounted outside of said narrowed section, said ring defining a maximal diameter of said narrowed section.

23. A reducer according to any of claims 1-22, wherein different parts of said reducer have different degrees of resistance to deforming.

24. A reducer according to claim 23, wherein said narrowed section has a greater resistance to deformation than said flared section.

25. A reducer according to claim 23, wherein a rim area of said flared section has a greater resistance to deformation than an adjacent part of said flared section.

26. A reducer according to any of the preceding claims, wherein said narrowed section has a cross-sectional area of less than 40% of a maximum cross-sectional area of said flared section.

27. A reducer according to any of claims 1-26, wherein said narrowed section has a cross-sectional area of less than 20% of a maximum cross-sectional area of said flared section.

28. A reducer according to any of claims 1-27, wherein said flared section has an axial extent of between 4 mm and 10 mm.

29. A reducer according to any of claims 1-28, wherein said at least one flared section comprises at least two flared sections (200, 202).

30. A reducer according to any of claims 1-29, wherein said reducer describes an hourglass figure.

31. A reducer according to any of claims 1-30, wherein said flared section is dense, to reduce blood flow therethrough.

32. A reducer according to claim 31, wherein the dense mesh fills at least 80% of the surface of the reducer.

33. A reducer according to any of claims 1-32, wherein said flared section is coated, to reduce blood flow therethrough.

34. A reducer according to any of claims 1-33, wherein said reducer is formed of a soft material, to reduce contact force against an enclosing vessel wall.

35. A reducer according to any of claims 1-34, wherein said reducer has an outside surface adapted to attach to a wall of a vein.

36. A reducer according to any of claims 1-35, wherein said narrowed section comprises a valve.

37. A reducer according to any of the preceding claims, wherein the reducer is asymmetric, in accordance with a configuration of a coronary sinus.

38. A reducer according to any of the preceding claims, wherein said reducer is not symmetric around its axis.

39. A reducer according to any of the preceding claims, wherein said reducer defines a curved blood flow passage.

40. A reducer according to any of the preceding claims, wherein said axial lumen is configured to allow passage therethrough of at least 40% of blood passing through a blood vessel in which the reducer is positioned.

41. A reducer according to any of the preceding claims, wherein the reducer is formed of a material and has a geometry that does not cause coagulation of blood in its vicinity.

## Patentansprüche

1. Reduzierstück (100) zum Einsetzen in ein Blutgefäß mit: mindestens einem verengten Abschnitt (204), der ein Axiallumen bildet, Blutdurchfluß ermöglicht und einen ersten Durchmesser hat; und mindestens einem aufgeweiteten Abschnitt (200), wobei das Reduzierstück geeignet ist, aus einer ersten Konfiguration, in der das Reduzierstück nicht expandiert ist, in die expandierte Konfiguration verformt zu werden, **dadurch gekennzeichnet, daß** der mindestens eine aufgeweitete Abschnitt einen größten Durchmesser hat, der mindestens 20 % größer als der erste Durchmesser in einer expandierten Konfiguration ist, und der mindestens eine verengte Abschnitt einen Innendurchmesser und eine Länge hat, die geeignet sind, eine gewünschte Fließdynamik und/oder Druckdifferenz über das Reduzierstück zu erreichen.

2. Reduzierstück nach Anspruch 1, wobei der größte Durchmesser des mindestens einen aufgeweiteten Abschnitts eine ausreichende Größe hat, um im wesentlichen einen gesamten Querschnitt einer Koronarvene eines Erwachsenen zu belegen.

3. Reduzierstück nach Anspruch 1 oder Anspruch 2, wobei der größte Durchmesser des mindestens einen aufgeweiteten Abschnitts eine ausreichende Größe hat, um im wesentlichen einen gesamten Querschnitt eines Koronarsinus eines Erwachsenen zu belegen.

4. Reduzierstück nach einem der Ansprüche 1 bis 3, wobei das Reduzierstück geeignet ist, bei Einsetzen in einen Koronarsinus eine intramyokardiale Perfusion zu erhöhen.

5. Reduzierstück nach einem der Ansprüche 1 bis 4, wobei der aufgeweitete Abschnitt mindestens einen Bereich (208) aufweist, der geeignet ist, eine Wand einer Vene zu kontaktieren.

6. Reduzierstück nach Anspruch 5, wobei der Bereich groß genug hergestellt ist, um Beschädigung der Wand zu verhindern.

7. Reduzierstück nach einem der Ansprüche 1 bis 6, wobei der aufgeweitete Abschnitt eine Außenkante (402) hat.

8. Reduzierstück nach Anspruch 7, wobei die Außenkante auf einer einzelnen Ebene liegt.

9. Reduzierstück nach Anspruch 7 oder Anspruch 8, wobei die Außenkante durch mehrere längliche Abschnitte (340, 342, 348)/(440, 446) gebildet ist, die zusammenwirken, um einen maximalen Rand (402) für das Reduzierstück zu bilden.

10. Reduzierstück nach einem der Ansprüche 7 bis 9, wobei die Außenkante glatt ist.

11. Reduzierstück nach einem der Ansprüche 7 bis 10, wobei die Außenkante zu einer Achse des Reduzierstücks nach innen gekrümmt ist.

12. Reduzierstück nach einem der Ansprüche 7 bis 11, wobei die Außenkante mit einem weichen Material beschichtet ist.

13. Reduzierstück nach einem der vorstehenden Ansprüche, wobei der verengte Abschnitt ein Ringsegment mit einer anderen Oberflächengestaltung als der aufgeweitete Abschnitt aufweist.

14. Reduzierstück nach einem der vorstehenden Ansprüche, wobei der verengte Abschnitt einen massiven Ring aufweist.

15. Reduzierstück nach einem der vorstehenden Ansprüche, wobei der verengte Abschnitt eine Anordnung aus Zellenelementen aufweist.

16. Reduzierstück nach einem der Ansprüche 1 bis 15, wobei der aufgeweitete Abschnitt in die expandierte Konfiguration plastisch verformbar ist.

17. Reduzierstück nach einem der Ansprüche 1 bis 15, wobei der aufgeweitete Abschnitt in die expandierte Konfiguration selbstexpandierend ist.

18. Reduzierstück nach einem der Ansprüche 1 bis 17, wobei der verengte Abschnitt geeignet ist, selbst in die expandierte Konfiguration zu expandieren.

19. Reduzierstück nach einem der Ansprüche 1 bis 18, wobei der verengte Abschnitt in die expandierte Konfiguration plastisch verformbar ist.

20. Reduzierstück nach einem der Ansprüche 1 bis 18, wobei der verengte Abschnitt geeignet ist, nicht zu expandieren, wenn das Reduzierstück aus der ersten Konfiguration in die expandierte Konfiguration expandiert.

21. Reduzierstück nach einem der Ansprüche 1 bis 20, wobei der verengte Abschnitt weiter expandierbar ist, nachdem sich das Reduzierstück in der expandierten Konfiguration befindet.

22. Reduzierstück nach einem der Ansprüche 1 bis 21 mit einem Ring, der außerhalb des verengten Abschnitts angeordnet ist, wobei der Ring einen maximalen Durchmesser des verengten Abschnitts bildet.

23. Reduzierstück nach einem der Ansprüche 1 bis 22, wobei unterschiedliche Teile des Reduzierstücks unterschiedliche Widerstandsgrade gegen Verformung haben.

24. Reduzierstück nach Anspruch 23, wobei der verengte Abschnitt einen größeren Verformungswiderstand als der aufgeweitete Abschnitt hat.

25. Reduzierstück nach Anspruch 23, wobei ein Randbereich des aufgeweiteten Abschnitts einen größeren Verformungswiderstand als ein benachbarter Teil des aufgeweiteten Abschnitts hat.

26. Reduzierstück nach einem der vorstehenden Ansprüche, wobei der verengte Abschnitt eine Querschnittfläche unter 40 % einer maximalen Querschnittfläche des aufgeweiteten Abschnitts hat.

27. Reduzierstück nach einem der Ansprüche 1 bis 26, wobei der verengte Abschnitt eine Querschnittfläche unter 20 % einer maximalen Querschnittfläche des aufgeweiteten Abschnitts hat.

28. Reduzierstück nach einem der Ansprüche 1 bis 27, wobei der aufgeweitete Abschnitt eine Axialausdehnung zwischen 4 mm und 10 mm hat.

29. Reduzierstück nach einem der Ansprüche 1 bis 28, wobei der mindestens eine aufgeweitete Abschnitt mindestens zwei aufgeweitete Abschnitte (200, 202) aufweist.

30. Reduzierstück nach einem der Ansprüche 1 bis 29, wobei das Reduzierstück eine Sanduhrfigur beschreibt.

31. Reduzierstück nach einem der Ansprüche 1 bis 30, wobei der aufgeweitete Abschnitt dicht ist, um Blutdurchfluß zu reduzieren.

32. Reduzierstück nach Anspruch 31, wobei das dichte Maschenmaterial mindestens 80 % der Oberfläche des Reduzierstücks füllt.

33. Reduzierstück nach einem der Ansprüche 1 bis 32, wobei der aufgeweitete Abschnitt beschichtet ist, um Blutdurchfluß zu reduzieren.

34. Reduzierstück nach einem der Ansprüche 1 bis 33, wobei das Reduzierstück aus einem weichen Material gebildet ist, um Kontaktkraft an einer umschließenden Gefäßwand zu reduzieren.

35. Reduzierstück nach einem der Ansprüche 1 bis 34, wobei das Reduzierstück eine Außenfläche hat, die geeignet ist, sich an einer Wand einer Vene anzulagern.

36. Reduzierstück nach einem der Ansprüche 1 bis 35, wobei der verengte Abschnitt ein Ventil aufweist.

37. Reduzierstück nach einem der vorstehenden Ansprüche, wobei das Reduzierstück in Übereinstimmung mit einer Konfiguration eines Koronarsinus asymmetrisch ist.

38. Reduzierstück nach einem der vorstehenden Ansprüche, wobei das Reduzierstück um seine Achse nicht symmetrisch ist.

39. Reduzierstück nach einem der vorstehenden Ansprüche, wobei das Reduzierstück einen gekrümmten Blutflußdurchgang bildet.

40. Reduzierstück nach einem der vorstehenden Ansprüche, wobei das Axiallumen so konfiguriert ist, daß es den Durchgang von mindestens 40 % von Blut ermöglicht, das ein Blutgefäß durchfließt, in dem das Reduzierstück positioniert ist.

41. Reduzierstück nach einem der vorstehenden Ansprüche, wobei das Reduzierstück aus einem Material gebildet ist und eine Geometrie hat, das (die) keine Blutgerinnung in seiner Umgebung verursacht.

## Revendications

1. Réducteur (100) à insérer dans un vaisseau sanguin, comprenant : au moins une section rétrécie (204) définissant une lumière axiale, permettant l'écoulement de sang à travers elle, ayant un premier diamètre, et au moins une section évasée (200), où ledit réducteur est adapté pour être déformé d'une première configuration, dans laquelle ledit réducteur est non dilaté, à la configuration dilatée, **caractérisé en ce que** ladite au moins une section évasée a un diamètre supérieur d'au moins 20 % audit premier diamètre, dans une configuration dilatée, et ladite au moins une section rétrécie a un diamètre interne et une longueur adaptés pour atteindre une dynamique de flux et/ou un différentiel de pression souhaités à travers le réducteur.

2. Réducteur selon la revendication 1, dans lequel le diamètre le plus grand de ladite au moins une section évasée a une taille suffisante pour occuper sensiblement une section transversale totale d'une veine coronaire d'un adulte.

3. Réducteur selon la revendication 1 ou 2, dans lequel le diamètre le plus grand de ladite au moins une section évasée a une taille suffisante pour occuper sensiblement une section transversale totale d'un sinus coronaire d'un adulte.

4. Réducteur selon l'une quelconque des revendications 1-3, dans lequel ledit réducteur est adapté pour augmenter une irrigation intra-myocardique quand il est inséré dans un sinus coronaire.

5. Réducteur selon l'une quelconque des revendications 1-4, dans lequel ladite section évasée comprend au moins une zone (208) adaptée pour entrer en contact avec la paroi d'une veine.

6. Réducteur selon la revendication 5, dans lequel ladite zone est réalisée de façon à être suffisamment grande pour empêcher des dommages à la paroi.

7. Réducteur selon l'une quelconque des revendications 1-6, dans lequel ladite section évasée a un bord extérieur (402).

8. Réducteur selon la revendication 7, dans lequel ledit bord extérieur se trouve sur un plan unique.

9. Réducteur selon la revendication 7 ou la revendication 8, dans lequel ledit bord extérieur est défini par une pluralité de sections allongées (340, 342, 348)/(440, 446) qui coopèrent pour définir un rebord maximum pour ledit réducteur.

10. Réducteur selon l'une quelconque des revendications 7-9, dans lequel ledit bord extérieur est lisse.

11. Réducteur selon l'une quelconque des revendications 7-10, dans lequel ledit bord extérieur est incurvé vers l'intérieur vers un axe dudit réducteur.

12. Réducteur selon l'une quelconque des revendications 7-11, dans lequel ledit bord extérieur est revêtu d'un matériau doux.

13. Réducteur selon l'une quelconque des revendications précédentes, dans lequel ladite section rétrécie comprend un segment en anneau ayant un modèle de surface différent depuis la section évasée.

14. Réducteur selon l'une quelconque des revendications précédentes, dans lequel ladite section rétrécie comprend un anneau solide.

15. Réducteur selon l'une quelconque des revendications précédentes, dans lequel ladite section rétrécie comprend une rangée d'éléments cellulaires.

16. Réducteur selon l'une quelconque des revendications 1-15, dans lequel ladite section évasée est déformable plastiquement pour atteindre ladite configuration dilatée.

17. Réducteur selon l'une quelconque des revendications 1-15, dans lequel ladite section évasée est auto-expansible pour atteindre ladite configuration dilatée.

18. Réducteur selon l'une quelconque des revendications 1-17, dans lequel ladite section rétrécie est adaptée pour s'auto-dilater afin d'atteindre ladite configuration dilatée.

19. Réducteur selon l'une quelconque des revendications 1-18, dans lequel ladite section rétrécie est déformable plastiquement jusqu'à ladite configuration dilatée.

20. Réducteur selon l'une quelconque des revendications 1-18, dans lequel ladite section rétrécie est adaptée pour ne pas se dilater quand le réducteur se dilate de la première configuration à la configuration dilatée.

21. Réducteur selon l'une quelconque des revendications 1-20, dans lequel ladite section rétrécie est ultérieurement dilatable après que ledit réducteur a atteint ladite configuration dilatée.

22. Réducteur selon l'une quelconque des revendications 1-21, comprenant un anneau monté à l'extérieur de ladite section rétrécie, ledit anneau définissant un diamètre maximal de ladite section rétrécie.

23. Réducteur selon l'une quelconque des revendications 1-22, dans lequel les différentes parties dudit réducteur ont des degrés différents de résistance à la déformation.

24. Réducteur selon la revendication 23, dans lequel ladite section rétrécie a une résistance à la déformation plus importante que ladite section évasée.

25. Réducteur selon la revendication 23, dans lequel une zone de rebord de ladite section évasée a une résistance à la déformation plus importante qu'une partie adjacente de ladite section évasée.

26. Réducteur selon l'une quelconque des revendications précédentes, dans lequel ladite section rétrécie a une superficie transversale inférieure de 40 % par rapport à une superficie transversale maximale de ladite section évasée.

27. Réducteur selon l'une quelconque des revendications 1-26, dans lequel ladite section rétrécie a une superficie transversale inférieure de 20 % par rapport à une superficie transversale maximale de ladite section évasée.

28. Réducteur selon l'une quelconque des revendications 1-27, dans lequel ladite section évasée a une ampleur axiale comprise entre 4 mm et 10 mm.

29. Réducteur selon l'une quelconque des revendications 1-28, dans lequel ladite au moins une section évasée comprend au moins deux sections évasées (200, 202).

30. Réducteur selon l'une quelconque des revendications 1-29, dans lequel ledit réducteur est représenté sous la forme d'un sablier.

31. Réducteur selon l'une quelconque des revendications 1-30, dans lequel ladite section évasée est dense, pour réduire l'écoulement de sang à travers elle.

32. Réducteur selon la revendication 31, dans lequel la maille dense remplit au moins 80 % de la surface du réducteur.

33. Réducteur selon l'une quelconque des revendications 1-32, dans lequel ladite section évasée est revêtue, pour réduire l'écoulement de sang à travers elle.

34. Réducteur selon l'une quelconque des revendications 1-33, dans lequel ledit réducteur est réalisé en matériau doux, pour réduire la force de contact contre une paroi du vaisseau qui le contient.

35. Réducteur selon l'une quelconque des revendications 1-34, dans lequel ledit réducteur a une surface extérieure adaptée pour être fixée à une paroi d'une veine.

36. Réducteur selon l'une quelconque des revendications 1-35, dans lequel ladite section rétrécie comprend une valve.

37. Réducteur selon l'une quelconque des revendications précédentes, dans lequel ledit réducteur est asymétrique, conformément à une configuration d'un sinus coronaire.

38. Réducteur selon l'une quelconque des revendications précédentes, dans lequel ledit réducteur n'est pas symétrique autour de son axe.

39. Réducteur selon l'une quelconque des revendications précédentes, dans lequel ledit réducteur définit un passage de flux sanguin incurvé.

40. Réducteur selon l'une quelconque des revendications précédentes, dans lequel ladite lumière axiale est configurée pour permettre le passage à travers elle d'au moins 40 % du sang passant à travers un vaisseau sanguin, dans lequel le réducteur est placé.

41. Réducteur selon l'une quelconque des revendications précédentes, dans lequel le réducteur est constitué d'un matériau et a une géométrie qui ne provoque pas la coagulation du sang à proximité.
